# FASCICULE DE BREVET EUROPEEN

(11) **EP 2 891 876 B1**
(45) Date de publication et mention de la délivrance du brevet: **18.03.2020**
(21) Numéro de dépôt: 14198359.3
(22) Date de dépôt: 16.12.2014
(51) Int. Cl.: G01N 21/35, G01N 33/00, G01N 21/03, G02B 5/10, H05B 3/14, H05B 3/26, G01N 21/3504

(54) **Détecteur optique d'un gaz**
Optischer Detektor eines Gases
Optical gas detector

(30) Priorité: 07.01.2014 FR 1450069
(43) Date de publication de la demande: 08.07.2015
(73) Titulaire: COMMISSARIAT À L'ÉNERGIE ATOMIQUE ET AUX ÉNERGIES ALTERNATIVES, 75015 Paris (FR)
(72) Inventeur: Nicoletti, Sergio, 38650 SINARD (FR); Brun, Mickaël, 38320 EYBENS (FR); Gidon, Serge, 38140 LA MURETTE (FR)
(74) Mandataire: Cabinet Beaumont

(56) Documents cités:
- US-A1- 2007 242 720
- US-A1- 2012 267 532
- PIERRE BARRITAULT ET AL: "Mid-IR source based on a free-standing microhotplate for autonomous CO sensing in indoor applications", SENSORS AND ACTUATORS A, ELSEVIER SEQUOIA S.A., LAUSANNE, CH, vol. 172, no. 2, 21 septembre 2011 (2011-09-21), pages 379-385, XP028336715, ISSN: 0924-4247, DOI: 10.1016/J.SNA.2011.09.027 [extrait le 2011-10-10]

## Description

### Domaine

La présente demande concerne un détecteur optique de présence et éventuellement de teneur d'un gaz dans une atmosphère.

### Exposé de l'art antérieur

Il est connu d'utiliser des détecteurs optiques de présence d'un gaz, par exemple de dioxyde de carbone, de monoxyde de carbone, de méthane ou éventuellement de divers gaz toxiques tels que du xylène ou du toluène dégagés par les peintures. Il est à noter qu'un détecteur de présence d'un excès de dioxyde de carbone et/ou de monoxyde de carbone peut constituer un détecteur d'incendie.

On s'intéressera ici à des détecteurs optiques qui détectent la présence d'un gaz en mesurant l'absorption d'un faisceau lumineux à une ou plusieurs longueurs d'onde correspondant à une ou plusieurs raies d'absorption du gaz considéré. Dans de tels détecteurs, un émetteur de rayonnement produit un faisceau lumineux dans une plage de longueurs d'onde incluant la ou les longueurs d'onde de raies d'absorption caractéristiques du gaz à détecter. Un récepteur de rayonnement précédé d'un filtre à la longueur d'onde de la raie d'absorption à détecter indique l'absorption à cette longueur d'onde, et on peut en déduire la présence et la teneur du gaz considéré. Le ou les filtres peuvent correspondre à une alternance de couches minces diélectriques. Il peut aussi s'agir d'une alternance de bandes métalliques et isolantes dont le pas détermine la longueur d'onde de filtrage.

Pour que l'ensemble du système détecteur de gaz ait un encombrement réduit, on prévoit souvent que le faisceau lumineux se propageant entre l'émetteur et le récepteur fasse un ou plusieurs trajets en aller-retour par l'intermédiaire de miroirs, généralement des miroirs courbes.

Le document US2007242720 décrit une cellule optique ayant un axe optique traversant les centres de rayons de première et seconde surfaces réfléchissantes supportées par une structure support comprenant des première et seconde bases de support. La source de lumière et le détecteur sont éloignés de la deuxième base de support de sorte que la lumière émise par la source soit réfléchie dans la cellule optique au moins une fois entre les première et deuxième surfaces réfléchissantes avant d'atteindre le détecteur.

De façon générale, les systèmes détecteurs de gaz existants impliquant au moins un aller-retour de faisceau lumineux entre l'émetteur de rayonnement et le récepteur de rayonnement présentent l'inconvénient d'être relativement délicats à fabriquer. En effet, l'émetteur, le récepteur et les miroirs nécessitent un positionnement précis les uns par rapport aux autres.

### Résumé

Il existe un besoin pour un détecteur optique de gaz par absorption qui soit particulièrement simple à fabriquer.

On souhaite également que ce détecteur soit tolérant à des défauts de positionnement de l'émetteur, des miroirs et du récepteur les uns par rapport aux autres.

La présente invention est définie dans la revendication 1. Des modes de réalisation préférés sont définis dans les revendications dépendantes 2 à 6.

Selon un mode de réalisation, le miroir plan et le récepteur ou l'émetteur forment un ensemble unique comprenant une plaque support dont une partie centrale comprend le récepteur ou l'émetteur et dont la partie restante constitue le miroir plan.

Selon un mode de réalisation, le miroir plan comprend un revêtement réfléchissant métallique comprenant au moins deux parties isolées l'une de l'autre, chaque partie du revêtement étant en contact avec une piste métallique reliée à une borne du récepteur ou émetteur.

Selon un mode de réalisation, le récepteur ou l'émetteur est disposé sur une membrane suspendue au-dessus d'une cavité centrale de la plaque support.

Selon un mode de réalisation, le détecteur de gaz comprend en outre au moins une couche isolante comportant une portion centrale prolongée par des bras reliés au reste de ladite au moins une couche, la portion centrale de ladite au moins une couche constituant la membrane, le reste de ladite au moins une couche reposant sur la plaque support.

Selon un mode de réalisation, les pistes métalliques reposent sur les bras.

### Brève description des dessins

Ces caractéristiques et avantages, ainsi que d'autres, seront exposés en détail dans la description suivante de modes de réalisation particuliers faite à titre non limitatif en relation avec les figures jointes parmi lesquelles :
la figure 1 est une vue en coupe schématique d'un mode de réalisation d'un détecteur optique de gaz,
les figures 2A et 2B sont des vues schématiques d'un mode de réalisation d'un ensemble miroir plan-récepteur, et
les figures 3A à 3C, 4A à 4C, 5A à 5C et 6A et 6B sont des vues schématiques illustrant des étapes d'un mode de réalisation d'un procédé de fabrication d'un ensemble miroir plan - récepteur, les figures 3B et 3C, 4B et 4C, 5B et 5C, et 6B étant des vues en coupe selon les plans BB et CC des figures indexées A correspondantes.

Par souci de clarté, de mêmes éléments ont été désignés par de mêmes références aux différentes figures et, de plus, les diverses figures ne sont pas tracées à l'échelle.

### Description détaillée

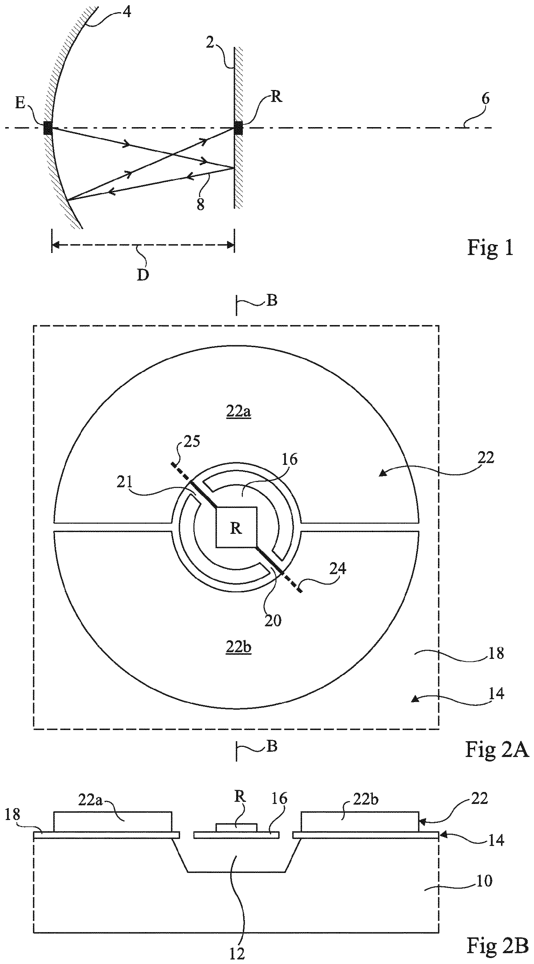

La figure 1 est une vue en coupe schématique d'un mode de réalisation d'un détecteur optique de gaz par absorption.

Le détecteur comprend un miroir plan 2 tourné vers un miroir sphérique concave 4 de rayon de courbure R_{C}, un émetteur de rayonnement E, et un récepteur de rayonnement R. L'axe optique 6 du système correspond à l'axe optique du miroir sphérique 4 et est orthogonal au miroir plan 2.

L'émetteur E est disposé au niveau de l'intersection du miroir sphérique 4 avec l'axe optique 6 et le récepteur R est disposé au niveau de l'intersection du miroir plan 2 et de l'axe optique 6. L'émetteur E est de préférence un émetteur peu ou non directionnel (émetteur divergent) tourné vers le miroir plan 2. L'émetteur E et le récepteur R sont liés respectivement aux miroirs 2 et 4 par des moyens non représentés en figure 1.

Les miroirs 2 et 4 sont disposés de sorte que la distance D séparant les intersections respectives des miroirs 2 et 4 avec l'axe optique 6 soit égale à 1 % près, voire à 5 % près, voire à 10 % près, aux trois quarts du rayon de courbure R_{C} du miroir sphérique 4 (D=0,75^{∗}R_{C}). Dans ces conditions, lorsqu'un faisceau, dont on a représenté un rayon 8, est émis par l'émetteur E, il est réfléchi par le miroir plan 2 puis par le miroir sphérique 4 avant d'atteindre le récepteur R. Le faisceau parcourt donc trois fois la cavité. Le grandissement du système est égal à 0,5, c'est-à-dire que l'image de l'émetteur sur le récepteur a une dimension moitié de celle de l'émetteur E.

Un détecteur de gaz comprenant des miroirs, tel que le détecteur de la figure 1, est bien adapté à la détection de gaz dont la ou les raies d'absorption sont dans l'infrarouge. L'émetteur peut alors être un filament chauffé à une température propre à émettre une quantité suffisante de rayonnement dans une plage de longueur d'onde incluant la raie d'absorption à détecter, par exemple une température comprise entre 350°C et 650°C pour une longueur d'onde de détection de 4,25 pm correspondant à une raie d'absorption du dioxyde de carbone.

A titre d'exemple, le récepteur R comprend un composant passif (résistance) ou un composant actif (diode ou transistor) dont les caractéristiques se modifient en fonction de leur échauffement dû à la réception de rayons infrarouges. Ainsi, le récepteur R peut être un capteur bolométrique ou pyrométrique, ou une thermopile.

Des simulations ont été effectuées par les inventeurs et montrent qu'un tel système est peu sensible à des déréglages. A titre d'exemple, lorsque l'on fait varier de 10 % la distance D entre les miroirs 2 et 4, l'image de l'émetteur est agrandie et déborde du récepteur ; l'énergie reçue diminue alors de 10 % seulement par rapport à l'énergie reçue dans le cas idéal où l'intégralité de l'image de l'émetteur occupe la surface du récepteur. Lorsque l'on incline de 0,05 ° ou que l'on déplace latéralement de 20 µm l'un des miroirs 2 ou 4 par rapport à l'axe optique 6, l'image de l'émetteur sur le récepteur est déplacée et l'énergie reçue sur le récepteur ne diminue également que de 10 % par rapport au cas idéal.

Cette tolérance de positionnement est notamment liée au fait que le récepteur R et l'émetteur E sont solidaires respectivement du miroir 2 et du miroir 4.

De plus, du fait que l'un des miroirs constituant le système optique du détecteur de gaz décrit précédemment est un miroir plan plutôt qu'un miroir courbe, le système est plus simple à fabriquer.

Les figures 2A et 2B représentent schématiquement un mode de réalisation d'un ensemble miroir plan - récepteur, la figure 2B étant une vue en coupe de la figure 2A selon le plan de coupe BB. L'ensemble comprend :
- une plaque support 10 munie d'une cavité 12 sur l'une de ses faces ;
- une couche ou un ensemble de couches 14, généralement isolantes, comprenant une portion centrale 16, une portion extérieure 18, et des bras 20 et 21 reliant entre elles les portions 16 et 18 ; la portion 16 constitue une membrane au-dessus de la cavité 12 et la portion 18 revêt la plaque support 10 autour de la cavité 12 ;
- un revêtement réfléchissant 22 revêtant la plus grande partie de la portion 18 de la couche 14 ; couramment le revêtement est métallique, par exemple une couche d'or ;
- un récepteur R solidaire de la membrane 16 ; et
- des pistes métalliques 24 et 25 pour connecter le récepteur.

Dans l'exemple représenté, le revêtement réfléchissant 22 a la forme d'un disque divisé en deux demi-disques 22a et 22b séparés et isolés l'un de l'autre. Les pistes métalliques 24 et 25 reposent respectivement sur le bras 20 et sur le bras 21. Les pistes métalliques 25 et 24 s'étendent à partir du récepteur R, respectivement sous la partie 22a et sous la partie 22b du revêtement 22. Ainsi le revêtement 22, en plus de constituer la surface réfléchissante du miroir plan 2, est utilisé pour connecter électriquement le récepteur R, les parties 22a et 22b constituant des électrodes.

Avantageusement, les pertes thermiques au niveau du récepteur R sont limitées du fait que la membrane 16 supportant le récepteur est suspendue au-dessus d'une cavité.

Le récepteur R et le miroir plan 2 constituent un ensemble permettant de supprimer l'étape d'assemblage de ces deux éléments et d'assurer un positionnement précis et stable du récepteur R par rapport au miroir plan 2.

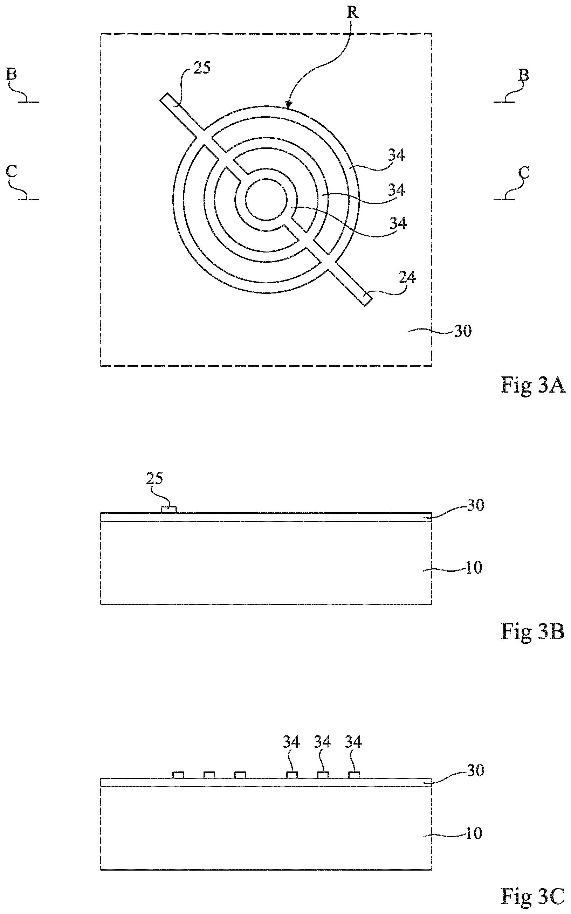

Les figures 3A à 6B sont des vues schématiques d'un exemple de la partie centrale d'un ensemble miroir plan - récepteur à différentes étapes d'un exemple de procédé de fabrication.

La figure 3A est une vue schématique et de dessus de la partie centrale de l'ensemble miroir plan - récepteur après des premières étapes, les figures 3B et 3C étant des vues en coupe selon les plans BB et CC respectivement. Ces figures représentent une plaque support 10, par exemple en silicium, après une étape de dépôt d'une couche isolante 30, par exemple en SiO₂ ou Si₃N₄, suivie d'une étape de dépôt d'une couche métallique. La couche métallique a été gravée pour former des anneaux métalliques concentriques 34 reliés entre eux par des pistes métalliques rectilignes 24 et 25 reposant sur la couche isolante 30. L'ensemble des anneaux concentriques 34 et les pistes métalliques 24 et 25 forment un récepteur de rayonnement R. Les anneaux métalliques 34 et les pistes métalliques 24 et 25 sont, par exemple, en platine ou en nitrure de titane. Ainsi, la tension entre les pistes 24 et 25 dépend de la résistance des anneaux métalliques. Cette résistance dépend de la température et constitue une indication de l'intensité d'un rayonnement frappant les anneaux métalliques 34.

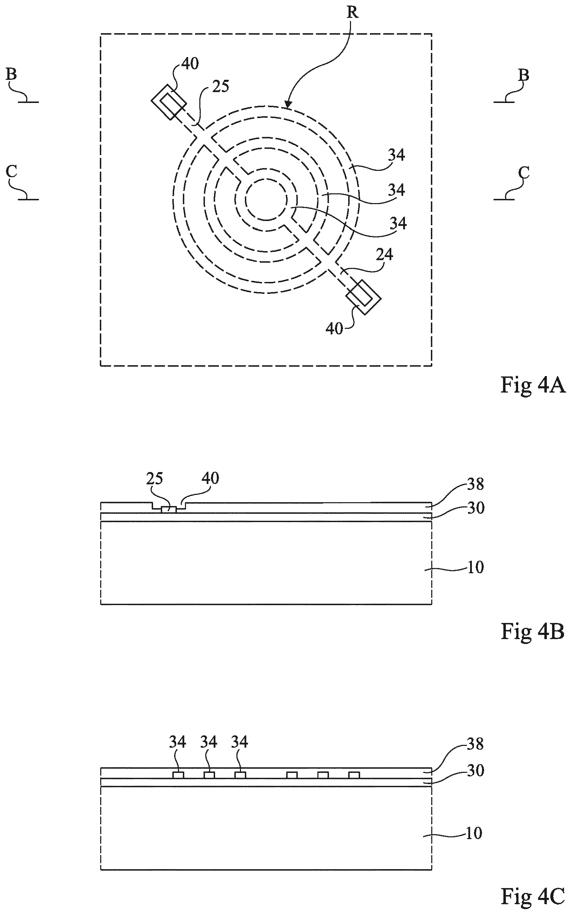

Les figures 4B et 4C sont des vues en coupe de la figure 4A, respectivement selon les plans BB et CC. Les figures 4A à 4C représentent schématiquement la structure des figures 3A à 3C après les étapes successives suivantes :
- dépôt d'une couche 38 isolante, par exemple en SiO₂ ou Si₃N₄, et
- gravure de la couche 38 pour former des ouvertures 40 découvrant les extrémités des pistes métalliques 24 et 25 du récepteur R.

Dans la figure 4A et dans les figures suivantes, les parties du récepteur de rayonnement R recouvertes de la couche 38 sont représentées en pointillés.

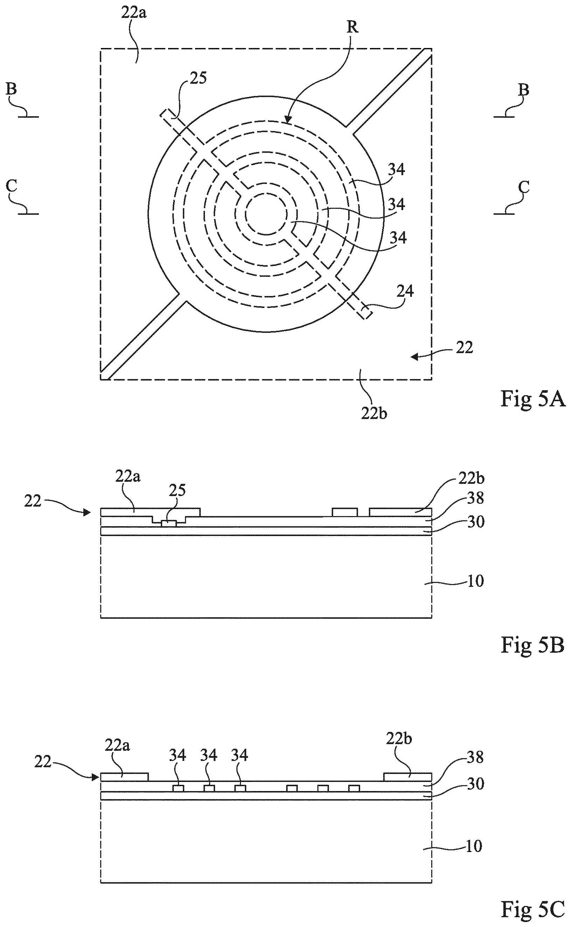

Les figures 5B et 5C sont des vues en coupe de la figure 5A, respectivement selon les plans BB et CC. Les figures 5A à 5C représentent schématiquement la structure des figures 4A à 4C après les étapes successives suivantes :
- dépôt d'un revêtement réfléchissant 22, et
- gravure du revêtement 22 pour découvrir une portion centrale de la couche 38 et pour séparer le revêtement 22 en deux parties 22a et 22b.

Comme on l'a indiqué précédemment, le revêtement 22 est de préférence une couche métallique, chacune des deux parties 22a et 22b du revêtement 22 étant reliée respectivement à l'extrémité de la piste 25 et à l'extrémité de la piste 24 du récepteur R pour connecter électriquement ce dernier.

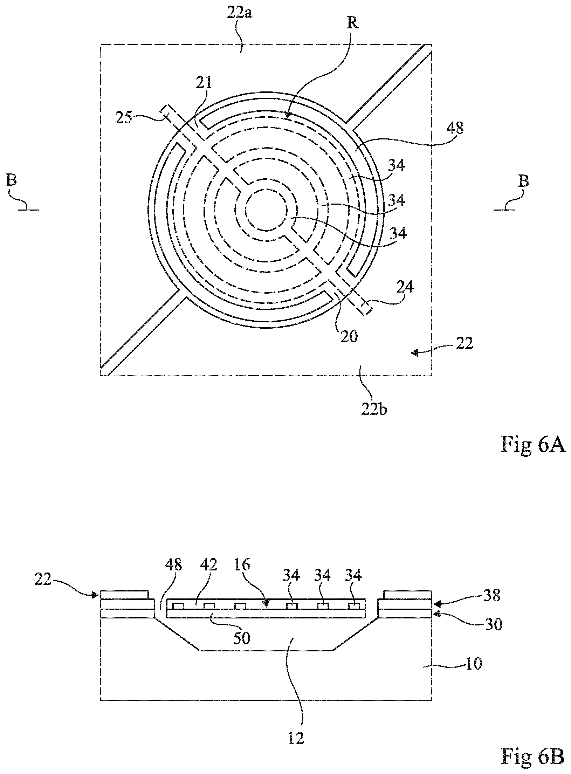

La figure 6B est une vue en coupe de la figure 6A selon le plan BB. Les figures 6A et 6B représentent schématiquement la structure des figures 5A à 5C après les étapes successives suivantes :
- gravure des couches 30 et 38 pour former une ouverture 48 autour de l'ensemble des anneaux 34 du récepteur R,
- gravure de la plaque support 10 depuis l'ouverture 48 pour former une cavité centrale 12 sous une membrane 16 portant le récepteur R.

La membrane est constituée d'une portion centrale 42 de la couche 38 et d'une portion centrale 50 de la couche 30, la portion centrale 50 étant en regard de la portion centrale 42. Les portions centrales 42 et 50 se prolongent par des bras 20 et 21 visibles en figure 6A. Les bras 20 et 21 portent une partie des pistes 24 et 25, respectivement. Ces bras relient les portions centrales 50 et 42 des couches 30 et 38 au reste des couches 30 et 38 reposant sur la plaque support 10. Les bras 20 et 21 permettent de maintenir en place la membrane 16.

A titre d'exemple, si on veut que le chemin optique entre l'émetteur et le récepteur soit de 8 cm, la distance D entre les miroirs 2 et 4 sera égale à environ le tiers de cette valeur, soit D=2,67 cm. Les dimensions de l'émetteur E, du récepteur R et des miroirs 2 et 4 seront choisies par l'homme de l'art en fonction de l'encombrement souhaité ainsi qu'en fonction des performances souhaitées du détecteur. Par exemple, on pourra choisir un diamètre de l'émetteur égal à 150 µm et un diamètre du récepteur égal à 75 pm. On pourra aussi choisir pour les miroirs plan et sphérique des diamètres égaux à 0,45 cm et 0,9 cm, respectivement. Le capteur de gaz ainsi obtenu est très compact.

Des modes de réalisation particuliers ont été décrits. Diverses variantes et modifications apparaîtront à l'homme de l'art.

Le récepteur peut être divisé en plusieurs récepteurs élémentaires, chacun étant associé à un filtre spécifique. Les différents récepteurs élémentaires permettent de fournir une indication de référence et/ou de détecter plusieurs gaz différents. La référence sert notamment à tenir compte de fluctuations environnementales et/ou de fluctuations d'intensité de l'émetteur.

L'homme de l'art pourra modifier l'ordre des étapes et/ou ajouter ou supprimer des étapes dans le procédé de fabrication décrit précédemment.

Bien que l'on ait décrit un détecteur de gaz dont l'émetteur E est solidaire du miroir sphérique 4 et dont le récepteur R est solidaire du miroir plan 2, en raison du principe du retour inverse de la lumière il est possible d'échanger les positions de l'émetteur E et du récepteur R. Le récepteur R est alors disposé au niveau du miroir sphérique 4, l'émetteur E étant disposé sur le miroir plan 2. Dans ce cas, le grandissement du système est égal à 2. On choisira de disposer l'émetteur ou le récepteur du côté miroir sphérique et de disposer le récepteur ou l'émetteur du côté du miroir plan en fonction d'impératifs spécifiques de fabrication. Ce choix sera également dicté par des contraintes dimensionnelles. En effet le dispositif (récepteur ou émetteur) disposé au niveau du miroir plan est deux fois plus petit que le dispositif au niveau du miroir sphérique.

Enfin, bien que l'on ait décrit un détecteur de gaz dans lequel le récepteur est maintenu par une membrane au-dessus d'une cavité, l'homme de l'art notera que, selon le type d'émetteur ou de récepteur choisi, une telle cavité peut ne pas être utile.

## Revendications

1. Détecteur de gaz comprenant :
un miroir plan (2) ;
un miroir sphérique (4) concave ayant un rayon de courbure (RC) et un axe optique (6) ;
un émetteur (E) et un récepteur (R) de rayonnement,
dans lequel :
le miroir sphérique (4) est tourné vers le miroir plan (2) ;
l'axe optique (6) du miroir sphérique (4) est orthogonal au miroir plan ;
l'émetteur (E) ou le récepteur (R) est disposé au niveau du point d'intersection du miroir sphérique (4) et de l'axe optique (6) ;
le récepteur (R) ou l'émetteur (E) est disposé au niveau du point d'intersection du miroir plan (2) et de l'axe optique (6) ; et
lorsque qu'un faisceau est émis par l'émetteur (E), il est réfléchi par le miroir plan (2) puis par le miroir sphérique (4) avant d'atteindre le récepteur (R),
**caractérisé en ce que** l'émetteur (E) et le récepteur (R) sont respectivement liés aux miroirs plan (2) et sphérique (4) aux intersections desdits miroirs avec l'axe optique (6) de sorte à former une ensemble unique ; et
**en ce que** la distance (D) séparant lesdites intersections est égale à 0,75 fois le rayon de courbure du miroir sphérique, à 10 % près.

2. Détecteur selon la revendication 1, dans lequel le miroir plan (2) et le récepteur (R) ou l'émetteur (E) forment un ensemble unique comprenant une plaque support (10) dont une partie centrale comprend le récepteur (R) ou l'émetteur (E) et dont la partie restante constitue le miroir plan (2).

3. Détecteur selon la revendication 2, dans lequel le miroir plan (2) comprend un revêtement réfléchissant métallique (22) comprenant au moins deux parties (22a, 22b) isolées l'une de l'autre, chaque partie du revêtement étant en contact avec une piste métallique (24, 25) reliée à une borne du récepteur (R) ou émetteur (E).

4. Détecteur selon la revendication 2 ou 3, dans lequel le récepteur (R) ou l'émetteur (E) est disposé sur une membrane (16) suspendue au-dessus d'une cavité centrale (12) de la plaque support (10).

5. Détecteur selon la revendication 4, comprenant en outre au moins une couche isolante (14 ; 30, 38) comportant une portion centrale (16 ; 42, 50) prolongée par des bras (20, 21) reliés au reste de ladite au moins une couche, la portion centrale de ladite au moins une couche constituant la membrane (16), le reste de ladite au moins une couche reposant sur la plaque support (10) .

6. Détecteur selon la revendication 5, dans lequel les pistes métalliques (24, 25) reposent sur les bras (20, 21).

## Patentansprüche

1. Ein Gasdetektor, der Folgendes aufweist:
einen Planspiegel (2);
einen konkaven sphärischen Spiegel (4) mit einem Krümmungsradius (RC) und einer optischen Achse (6);
einen Strahlungssender (E) und einen Strahlungsempfänger (R);
wobei:
der sphärische Spiegel (4) dem Planspiegel (2) zugewandt ist;
die optische Achse (6) des sphärischen Spiegels (4) orthogonal zu dem Planspiegel ist;
der Sender (E) oder der Empfänger (R) im Schnittpunkt des sphärischen Spiegels (4) und der optischen Achse (6) angeordnet ist;
der Empfänger (R) oder der Sender (E) im Schnittpunkt des Planspiegels (2) und der optischen Achse (6) angeordnet ist; und
wenn ein Strahl vom Sender (E) ausgesendet wird, der Strahl vom Planspiegel (2) und dann vom sphärischen Spiegel (4) reflektiert wird, bevor er den Empfänger (R) erreicht,
**dadurch gekennzeichnet, dass** der Sender (E) und der Empfänger (R) jeweils mit dem Planspiegel (2) und dem sphärischen Spiegel (4) an den Schnittpunkten der genannten Spiegel mit der optischen Achse (6) verbunden sind, um eine Einheit zu bilden; und
dass der Abstand (D) zwischen dem Planspiegel und dem sphärischen Spiegel gleich dem 0,75-fachen Krümmungsradius des sphärischen Spiegels ist, mit einer Genauigkeit von 10 %.

2. Detektor nach Anspruch 1, wobei der Planspiegel (2) und der Empfänger (R) oder der Sender (E) eine Einheit bilden, die eine Trägerplatte (10) aufweist, die einen zentralen Teil mit dem Empfänger (R) oder dem Sender (E) und einen restlichen Teil, der den Planspiegel (2) bildet, aufweist.

3. Detektor nach Anspruch 2, wobei der Planspiegel (2) eine metallische reflektierende Beschichtung (22) mit mindestens zwei voneinander getrennten bzw. isolierten Abschnitten (22a, 22b) aufweist, wobei jeder Abschnitt der Beschichtung mit einer Metall-Leiterbahn (24, 25) in Kontakt steht, die mit einem Anschluss des Empfängers (R) oder des Senders (E) verbunden ist.

4. Detektor nach Anspruch 2 oder 3, wobei der Empfänger (R) oder der Sender (E) auf einer Membran (16) angeordnet ist, die über einem zentralen Hohlraum (12) der Trägerplatte (10) aufgehängt ist.

5. Detektor nach Anspruch 4, der ferner mindestens eine Isolierschicht (14; 30, 38) mit einem zentralen Abschnitt (16; 42, 50) aufweist, der durch Arme (20, 21) fortgesetzt wird, die mit dem Rest der mindestens einen Schicht verbunden sind, wobei der zentrale Abschnitt der mindestens einen Schicht die Membran (16) bildet und der Rest der mindestens einen Schicht auf der Trägerplatte (10) liegt.

6. Detektor nach Anspruch 5, wobei die Metall-Leiterbahnen (24, 25) auf den Armen (20, 21) liegen.

## Claims

1. A gas detector comprising:
a planar mirror (2);
a concave spherical mirror (4) having a and an optical axis (6)
a radiation emitter (E) and a radiation receiver (R),
wherein:
the spherical mirror (4) is facing the planar mirror (2) ;
the optical axis (6) of the spherical mirror (4) is orthogonal to the planar mirror;
the emitter (E) or the receiver (R) is arranged at the point of intersection of the spherical mirror (4) and of the optical axis (6);
the receiver (R) or the emitter (E) is arranged at the point of intersection of the planar mirror (2) and of the optical axis (6) ; and
when a beam is emitted by emitter (E), it is reflected by the planar mirror (2) and then by the spherical mirror (4) before reaching the receiver (R),
**characterized in that** the emitter (E) and the receiver (R) are respectively connected to the planar (2) and spherical (4) mirrors at the intersections of said mirrors with the optical axis (6) so as to form a single assembly; and
**in that** the distance (D) between the planar and spherical mirrors being equal to 0.75 times the radius of curvature of the spherical mirror, to within 10%.

2. The detector of claim 1, wherein the planar mirror (2) and the receiver (R) or the emitter (E) form a single assembly comprising a support plate (10) having a central portion comprising the receiver (R) or the emitter (E) and having its remaining portion forming the planar mirror (2).

3. The detector of claim 2, wherein the planar mirror (2) comprises a metallic reflective coating (22) comprising at least two portions (22a, 22b) insulated from each other, each portion of the coating being in contact with a metal track (24, 25) connected to a terminal of the receiver (R) or the emitter (E) .

4. The detector of claim 2 or 3, wherein the receiver (R) or the emitter (E) is arranged on a membrane (16) suspended above a central cavity (12) of the support plate (10).

5. The detector of claim 4, further comprising at least one insulating layer (14; 30, 38) comprising a central portion (16; 42, 50) continued by arms (20, 21) connected to the rest of said at least one layer, the central portion of said at least one layer forming the membrane (16), the rest of said at least one layer being laid on the support plate (10).

6. The detector of claim 5, wherein the metal tracks (24, 25) rest on arms (20, 21).
